# EUROPÄISCHE PATENTSCHRIFT

(11) **EP 2 836 244 B1**
(45) Veröffentlichungstag und Bekanntmachung des Hinweises auf die Patenterteilung: **13.01.2016**
(21) Anmeldenummer: 13715981.0
(22) Anmeldetag: 11.04.2013
(51) Int. Cl.: A61L 27/42

(54) **IMPLANTAT AUS EINEM FASERVERBUNDWERKSTOFF**
IMPLANT MADE OF A FIBER COMPOSITE MATERIAL
IMPLANT EN MATÉRIAU COMPOSITE RENFORCÉ PAR DES FIBRES

(30) Priorität: 11.04.2012 DE 102012205888
(43) Veröffentlichungstag der Anmeldung: 18.02.2015
(73) Patentinhaber: InnoTERE GmbH, 01445 Radebeul (DE)
(72) Erfinder: NIES, Berthold, 64407 Fränkisch-Crumbach (DE); RÖSSLER, Sophie, 01156 Dresden (DE); STORCH, Sandra, 01468 Moritzburg (DE); CHERIF, Chokri, 01219 Dresden (DE); LAOURINE, Ezzeddine, 01069 Dresden (DE)
(74) Vertreter: Kailuweit & Uhlemann Patentanwälte Partnerschaft mbB
(86) Internationale Anmeldenummer: PCT/EP2013/057637
(87) Internationale Veröffentlichungsnummer: WO 2013/153185

(56) Entgegenhaltungen:
- WO-A2-2006/039733
- US-A- 4 230 455
- US-B2- 6 955 716

## Beschreibung

Die Erfindung betrifft ein Implantat, ein Knochenimplantat, welches ein erfindungsgemäßes Implantat enthält und ein Set zur Herstellung eines Implantats sowie deren Verwendungen. Ferner gibt die Erfindung ein Verfahren zur Herstellung eines erfindungsgemäßen Implantats an.

In der Orthopädie und Chirurgie besteht seit jeher ein Bedarf, Knochendefekte nach Frakturen, oder Amputation in Folge von Tumorerkrankungen oder Entzündungen mit Knochenmaterial oder einem dem Knochen ähnlichen Ersatz wieder aufzufüllen. CN 102085123 A offenbart hierbei ein Zweikomponentenimplantat, das aus einem nicht-gewebten Titan-Keil-Draht-Zylinder besteht, der mit einem Knochenersatzmaterial formschlüssig befüllt ist. Das Knochenersatzmaterial besteht aus grobkörnigem Calciumphosphatzement mit einem Masseanteil von 40-94 Gew.-% und einem Bindematerial mit einem Masseanteil von 5 bis 29 Gew.-%. Die beiden Zylinderköpfe des nicht-gewebten Titandrahtzylinders sind durch biodegradable Gehäusekappen verschlossen, wodurch das natürliche Knochengewebe von den Zylinderenden in das Implantat einwachsen kann. Der nicht-gewebte Titan-Keil-Draht-Zylinder ist somit nicht in das Knochenersatzmaterial inkludiert, sondert dient lediglich als Füllbehältnis.

Für die Implantation, insbesondere die Implantation in den Knochen, sind Materialien mit hoher mechanischer Belastbarkeit erforderlich. Zur Verbesserung der mechanischen Eigenschaften von Polymethylmethacrylatzementen ist bekannt, in diese eine Maschenstruktur einzubringen. US 4,064,567 offenbart dazu ein Verbindungssystem zwischen Knochen und Implantat, das eine Maschenstruktur, beispielsweise aus nichtresorbierbaren Metallen, Plastik, Carbon oder resorbierbaren Fasern, wie Dacron, enthält. Die Maschenstruktur ist mit einem Polymethylmethacrylatzement befüllt und soll zur Verbesserung der Fixierung von Prothesen dienen und u.a. Bruchstellen vermeiden.

Es wird zudem angestrebt, in Implantaten Materialien einzusetzen, die das Einwachsen des natürlichen Knochengewebes fördern (also bioaktiv sind) und im Körper sukzessive abgebaut werden (also resorbierbar sind), um damit eine spätere Entfernung eines Implantats zu vermeiden. Als bioaktive und resorbierbare Materialien sind mineralische Knochenzemente, beispielsweise auf der Basis von Calciumphosphaten, verbreitet. Es ist möglich mineralische Knochenzemente mit hoher Druckfestigkeit zu erzeugen, jedoch können Implantate aus reinem mineralischen Knochenzement nur sehr gering auf Biegung beansprucht werden.

Zur Verbesserung der mechanischen Eigenschaften von Implantaten auf Basis mineralischer Knochenzemente ist bekannt, mineralische Knochenzemente resorbierbaren Polymerfasern zu verstärken. Dadurch können die mechanischen Eigenschaften der Implantate auf Basis mineralischer Knochenzemente verbessert werden, jedoch sind die Druckfestigkeiten und Biegefestigkeiten noch unzureichend.

US 6,955,716 B2 offenbart hierin einen Calciumphosphat-Knochenersatzstoff, der mit einem Massenanteil von mehr als 20 Gew.-% nichtmetallische, resorbierbare Fasern enthält. Die nichtmetallischen, resorbierbaren Fasern sind dabei hauptsächlich ausgewählt aus Polymerfasern, wie z.B. Vicrylfäden, polyglactin 910, Ethicon, Somerville, N.J., Panacryl⁽™⁾-Fäden, Ethicon, NJ oder aus keramischen Siliziumcarbidfasern.

Zur Verbesserung der mechanischen Eigenschaften von Implantaten auf Basis mineralischer Knochenzemente ist es bekannt, resorbierbaren mineralischen Knochenzement in offenzellige Metallstrukturen, wie Schäume oder Hohlkugelstrukturen aus Metall, zu füllen. Ein derartiges Knochenimplantat ist in WO 2008/064672 offenbart, wobei ein resorbierbarer mineralischer Knochenzement auf Basis von Calciumphosphaten und/oder Magnesiumphosphaten in einen metallischen Schaum oder eine metallische Hohlkugelstruktur zumindest teilweise formschlüssig eingefüllt ist. Dazu werden metallische Strukturen ausgewählt, deren Steifigkeit im Bereich des gesunden kortikalen menschlichen Knochens liegt. Durch die metallische Struktur wird bereits zum Zeitpunkt der Implantation eine hohe Druckfestigkeit des Implantats ermöglicht. Durch die Resorbierbarkeit des mineralischen Knochenzements wächst Knochengewebe sukzessive in das Implantat ein, wobei das verbleibende Metall mit der vergleichbaren Steifigkeit, wie der gesunde Knochen zurückbleibt. Nachteilig an den eingesetzten Metallschäumen oder metallischen Hohlkugelstrukturen ist es, dass die mechanischen Eigenschaften nur limitiert einstellbar sind. Eine gerichtete Einstellung von mechanischen Eigenschaften ist nicht möglich, vielmehr weisen diese metallischen Strukturen bei Belastung aus beliebigen Richtungen die gleichen Belastungseigenschaften auf.

WO 2011/157758 A2 offenbart ein Knochenimplantat, das aus einem resorbierbaren magnesiumhaltigen Werkstoff mit einer korrosionshemmenden Beschichtung aus Magnesium-Ammonium-Phosphat und einem mineralischen Knochenzement besteht. Der magnesiumhaltige Werkstoff kann dabei in Form eines zentral platzierten Drahtes und/oder diffus verteilter Späne vorliegen.

Es besteht daher Bedarf, Implantate mit einstellbaren Belastungseigenschaften bereitzustellen, die insbesondere eine hohe Biegefestigkeit und hohe Druckfestigkeit aufweisen.

Aufgabe der Erfindung ist es Materialien für die Herstellung von Knochenimplantaten mit verbesserten mechanischen Eigenschaften, insbesondere mit einstellbaren mechanischen Eigenschaften, bereitzustellen. Eine weitere Aufgabe der Erfindung ist es, Materialien für die Anwendung in Knochenimplantaten bereitzustellen, die leicht zu verarbeiten sowie flexibel einsetzbar sind und zur Erzielung verbesserter mechanischer Eigenschaften der Knochenimplantate geeignet sind.

Die Aufgabe wird erfindungsgemäß gelöst durch ein Implantat aus einem Faserverbundwerkstoff, welches metallische Langfasern und/oder metallische Endlosfasern und resorbierbaren mineralischen Knochenzement enthält.

Das Implantat aus einem Faserverbundwerkstoff enthält dazu:
**a)** resorbierbaren mineralischen Knochenzement als Matrixmaterial, in dem
b) verstärkende metallische Langfasern und/oder metallische Endlosfasern mit einem Aspektverhältnis von mindestens 100 : 1, in Form mindestens einer gerüstgebenden Faserstruktur, die Kontur des Implantates vorformenden Faserstruktur eingebracht sind.

Dabei ist die gerüstgebende Faserstruktur zur Aufnahme gerichteter Belastungen an das Implantat für die zu reparierende Defektstelle des Knochens oder Gelenkes angepasst.

Unter einem Implantat wird im Sinne der Erfindung ein zur Implantation geeignetes Material verstanden. Das erfindungsgemäße Implantat besteht aus einem Faserverbundwerkstoff, der metallische Langfasern und/oder metallische Endlosfasern sowie resorbierbaren mineralischen Knochenzement enthält. Neben den metallischen Fasern und dem resorbierbaren mineralischen Knochenzement können weitere, unten näher definierte, Bestandteile im Faserverbundwerkstoff enthalten sein. Erfindungsgemäß sind die metallischen Langfasern und/oder metallischen Endlosfasern in Form mindestens einer gerüstgebenden Faserstruktur in dem mineralischen Knochenzement inkludiert, wobei die metallischen Fasern vollständig von dem Knochenzement umgeben sind. Die dreidimensionale Form einer gerüstgebenden Faserstruktur ist vorzugsweise an die zu reparierenden Defektstelle des Knochens angepasst.

Durch den Einsatz von resorbierbaren mineralischen Knochenzementen werden diese nach der Implantation im Körper sukzessive abgebaut und das natürliche Knochengewebe kann die behandelte Knochendefektstelle schließen.

Mineralische Knochenzemente in Form eines in Wasser schwerlöslichen Festkörpers werden durch die Reaktion von mineralischem Knochenzementpulver und Wasser gebildet. Nach der Vermischung des mineralischen Knochenzementpulvers mit Wasser wird zunächst eine pastöse Masse gebildet, die sukzessive zu einem Festkörper aushärtet. Zur Erleichterung der Anmischung kann das mineralische Knochenzementpulver auch in einer wasserfreien Trägerflüssigkeit dispergiert vorliegen, und damit die Form einer wasserfreien pastösen Zubereitung annehmen, die nach Kontakt oder Vermischung mit Wasser zu einem schwerlöslichen Festkörper aushärtet. Im Sinne der Erfindung werden von der Bezeichnung "mineralische Knochenzemente" sämtliche der zuvor genannten Varianten umfasst, also sowohl das mineralische Knochenzementpulver (welches noch nicht mit Wasser reagiert hat), eine Dispersion des mineralischen Knochenzementpulvers in Wasser, einer wässrigen Lösung oder in einer wasserfreien, nicht-wässrigen Trägerflüssigkeit als auch der ausgehärtete Festkörper. In einem erfindungsgemäßen Implantat liegt der resorbierbare mineralische Knochenzement im Faserverbundwerkstoff damit in Form eines Festkörpers (der durch die Abbindereaktion eines mineralischen Knochenzementpulvers mit Wasser gebildet wurde) oder in pastöser Form (als Dispersion mineralischen Knochenzementpulvers in Wasser oder einer wässrigen Lösung, die sukzessive aushärtet, oder als Dispersion mineralischen Knochenzementpulvers in einer wasserfreien und nicht-wässrigen Trägerflüssigkeit) vor.

Der Begriff "mineralisches Knochenzementpulver" bezeichnet hierin hingegen ausschließlich ein noch nicht mit Wasser reagiertes Pulver, welches zur Ausbildung eines schwerlöslichen Festkörpers nach Reaktion mit Wasser geeignet ist.

Durch die verstärkenden metallischen Fasern in dem mineralischen Knochenzement als Matrixmaterial werden besonders vorteilhafte mechanische Eigenschaften im erhaltenen Verbundmaterial erzielt, die im Vergleich zu den mechanischen Eigenschaften der einzelnen Materialien höherwertig sind. Da metallische Fasern in Faserrichtung eine um ein vielfaches höhere Festigkeit aufweisen können, als dasselbe Metall in anderer Form, ist es möglich, Materialien mit hoher spezifischer Festigkeit (Verhältnis von Festigkeit zu Gewicht) herzustellen, was für den Einsatz als Implantat von besonderem Vorteil ist. Im Gegensatz zu soliden Materialien oder Schäumen weisen Fasern in unterschiedliche Richtungen unterschiedliche Belastungseigenschaften auf. So ist in den Faserverbundwerkstoffen bei Belastungen senkrecht zur Faser keine Steigerung der Festigkeit festzustellen, hingegen ist eine signifikante Festigkeit bei Belastungen in Faserrichtung erzielbar. Dadurch ist es möglich, Faserverbundwerkstoffe bereitzustellen, die Verstärkungselemente genau an den Stellen enthalten, an denen diese benötigt werden um gerichtete Belastungen aufzunehmen. Die in den mineralischen Knochenzement eingebrachte dreidimensionale Faserstruktur aus verstärkenden metallischen Fasern ist demnach eine dreidimensional ausgelegte Faserstruktur, die eine gerüstgebende Funktion übernimmt, wobei die Verstärkungselemente so ausgerichtet sind, dass sie den Belastungen des Knochens angepasst sind. Die textilen Strukturen sind dabei an die dreidimensionale Kontur des Implantats formstabil angepasst.

Eine gerüstgebende Faserstruktur ist im Sinne der Erfindung eine dreidimensionale Faserstruktur, die die Kontur des Implantats vorformt und bei der die Faserstruktur so ausgerichtet ist, dass sie den Belastungen des Implantats für die zu reparierende Defektstelle des Knochens oder Gelenkes angepasst ist.

Um eine authentische oder annähernd authentische Rekonstruktion einer Defektstelle zu ermöglichen, stellt die Faserstruktur ein zumindest teilweise dreidimensionales Abbild des Implantates für die zu reparierende Defektstelle des Knochens dar.

Mit den erfindungsgemäßen Implantaten können damit zum Beispiel:
- Augmentate als zylinderförmiger Implantate für Femurfrakturen oder als Zwischenwirbelimplantate für Wirbelfusionen und Wirbelkörperersatzimplantate
- Augmentate für den Schienbeinkopf für eine Knierevison
- Plattenförmige Elemente z.B. für Schulterblatt-, Schädelfrakturen oder Beckenfrakturen
hergestellt werden.

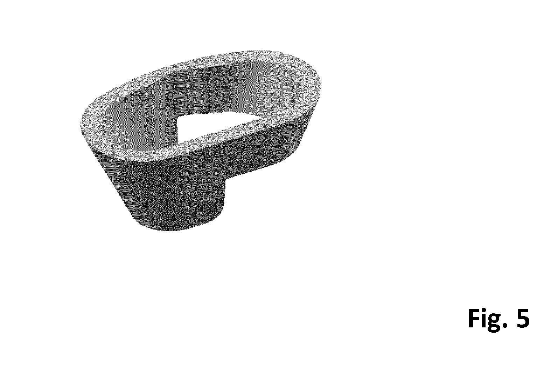

Fig. 5 zeigt ein Augmentat für den Schienbeinkopf für die Knierevision. Das in Fig. 5 dargestellte Augmentat ist ein konisch geformtes zylinderförmiges Teil mit einer Aussparung. Im Inneren befindet sich eine mehrlagige konisch geformte zylinderförmige gerüstgebende Faserstruktur aus metallische Langfasern und/oder metallische Endlosfasern, die in die Knochenzementmatrix eingebettet ist.

Durch die Vielfalt der möglichen durch metallische Fasern gebildeten Strukturen ist es möglich, Implantate beliebiger Abmessungen und Formen herzustellen. Es ist vorteilhaft möglich, gradierte Implantate bzw. Implantatmaterialien in Form von Faserverbundwerkstoffen herzustellen, die an gewünschten Stellen Verstärkungen (beispielsweise für die Befestigung eines Implantats am Knochen) und/oder an anderen Stellen Porositäten aufweisen (beispielsweise für das Einwachsen von Knochengewebe). Insbesondere ist es möglich, die verstärkenden Fasern im Faserverbundwerkstoff an den Stellen zu platzieren, an denen besonders hohe Zugkräfte auftreten können. Die Eigenschaften der Matrix und der verstärkenden metallischen Fasern können auf diese Weise optimal genutzt werden, indem die besonders gut auf Druck belastbare Matrix als Widerlager für die besonders stark auf Zug belastbaren metallischen Fasern und metallische Fasern enthaltende Faserhalbzeuge dient. Bei zylindrischen Implantaten konzentrieren sich die metallischen Fasern daher bevorzugt im äußeren Bereich. Bei flächigen Implantaten liegen die metallischen Fasern bevorzugt an beiden Außenseiten. Alternativ kann die Verstärkung mit metallischen Fasern bei vorhersehbarer Belastungsrichtung von einer Seite auf eine Seite beschränkt sein.

Für die Erfindung werden metallische Langfasern und/oder metallische Endlosfasern eingesetzt, die bevorzugt in Form eines Faserhalbzeugs vorliegen. Unter Langfasern werden Fasern einer Länge von 5 mm - 50 mm verstanden. Unter Endlosfasern werden Fasern einer Länge von mehr als 50 mm verstanden. Bevorzugt werden Fasern mit einer Länge von mindestens 10 mm eingesetzt, weiter bevorzugt mindestens 30 mm, besonders bevorzugt werden Endlosfasern eingesetzt. Bevorzugt weisen die Fasern ein Aspektverhältnis (Verhältnis von Länge zu Durchmesser der Faser) von mindestens 10 : 1, vorzugsweise mindestens 20 : 1, besonders bevorzugt von mindestens 100 : 1 auf. Eine besonders gute Verstärkung wird in einem erfindungsgemäßen Implantat durch ein möglichst großes Aspektverhältnis erzielt.

Die eingesetzten metallischen Langfasern und/oder metallischen Endlosfasern können eine beliebig geformte Querschnittsfläche aufweisen. Der Durchmesser der Faser beträgt dabei vorzugsweise maximal 3 mm, bevorzugt maximal 1 mm, besonders bevorzugt maximal 0,5 mm.

Die folgenden Ausgestaltungen sind für die Erfindung bevorzugt:

In einer bevorzugten Ausgestaltung der Erfindung sind die gerüstgebenden Faserstrukturen im Implantat in Vorzugsrichtungen ausgerichtet und/oder konzentrieren sich im äußeren Bereich des erfindungsgemäßen Implantats. Somit kann vorteilhaft die Aufnahme gerichteter Belastungen in der Form von Zugkräften, Druckkräften und/oder Biegekräften gewährleistet werden.

In einer Ausgestaltung der Erfindung liegen die gerüstgebenden Faserstrukturen in Form von Multifilamenten und/oder als Faserhalbzeug aus mindestens einer Lage vor.

Gelege, Geflechte oder Vliese können dabei aus mindestens einer Lage eines Geleges, Geflechts oder Vlieses bestehen. Bevorzugt sind mehrlagige textiltechnisch hergestellte Faserstrukturen, insbesondere Halbzeuge aus mehrlagigen Geweben, Gelegen oder Geflechten mit mindestens 2 Lagen, wobei zusätzliche Stehfäden in der Hauptbelastungsrichtung angeordnet sind. Auf Grund der mehrlagigen Anordnung textiltechnisch hergestellte Faserstrukturen, wird vorteilhaft eine höhere Dichte an metallischen Fasern im gradierten Implantat erzielt. Durch gezieltes Platzieren und Verteilen der textiltechnisch hergestellten Faserstrukturen kann so eine lokale oder ganzheitliche Verstärkung eines erfindungsgemäßen Implantats ermöglicht werden.

Bei der Anwendung von einzelnen Faserfilamenten oder beim Einsatz von Faserhalbzeugen liegen die metallischen Fasern in einer Ausgestaltung der Erfindung in Form länglicher Multifilamente, vorzugsweise gezwirnt, geflochten, als Fäden oder als Kordeln, vor. Dadurch sind Faserverbundwerkstoffe höherer Biegefestigkeit in Faserrichtung herstellbar. Besonders bevorzugt eignen sich dabei einzelne Fasern oder Faserbündel in Form von Stehfäden zur Erhöhung der Biege- und Torsionsfestigkeit.

In einer erfindungsgemäßen Ausgestaltung enthalten Faserstrukturen zwischen den metallischen Fasern Poren einer Größe von 100-2500 *µ*m.

Bevorzugt befinden sich dabei mehr als 80 Gew.-% der metallischen Fasern innerhalb eines Bereiches von 0,1 - 5 mm gemessen von der Außenseite des Implantats befinden.

Die metallischen Fasern können für die Verarbeitung in einem erfindungsgemäßen Implantat als Einzelfasern (Monofilamente), Faserbündel (Multifilamente) oder in Form daraus gefertigter Faserstrukturen (wie Kordeln oder Seile) oder in Form eines Faserhalbzeugs vorliegen. Bevorzugt ist aufgrund der einfacheren Handhabung die Form eines Faserhalbzeugs. Darin sind die einzelnen Fasern, vorzugsweise durch textiltechnische Verfahren (wie Weben, Stricken oder Flechten), zu einem Halbzeug zusammengefasst. Bevorzugte Formen von Faserhalbzeugen sind Gewebe, Gelege (inklusive Multiaxialgelege), Gestricke, Geflechte oder Vliese. In Geweben werden bevorzugt Endlosfasern verwoben. In Gelegen liegen die Fasern, bevorzugt Endlosfasern, parallel und gestreckt vor und werden durch eine Heftung zusammengehalten. Werden die Fasern nicht ausschließlich in der Ebene orientiert, liegt ein Multiaxialgelege vor. Besonders bevorzugte Faserhalbzeuge enthalten zwischen den Metallfaserfilamenten Poren einer Größe von 100-2500 µm (maximaler Abstand zweier benachbarter Fasern). Ein in einem erfindungsgemäßen Set enthaltenes Faserhalbzeug und ein in einem erfindungsgemäßen Verfahren eingesetztes Faserhalbzeug liegt bevorzugt in einer der vorgenannten Formen vor. Die dreidimensionale Form eines Faserhalbzeugs ist vorzugsweise an die Defektstelle des Knochens angepasst. Bevorzugt sind zylindrische oder plattenförmige Faserhalbzeuge, wobei darin bevorzugt die metallischen Fasern in den äußeren Bereichen des Zylinders oder der Platte angeordnet sind. Dabei befinden sich vorzugsweise mehr als 80 Gew.-% der metallischen Fasern innerhalb eines Bereiches von 0,1 - 5 mm gemessen von der Außenseite des erfindungsgemäßen Implantats. Die Ausrichtung der metallischen Langfasern und/oder Endlosfasern im Faserverbundwerkstoff beim Einsatz einzelner Filamente der Fasern kann in unterschiedlicher Weise erfolgen. In einer Ausführungsform sind die metallischen Langfasern und/oder Endlosfasern im Faserverbundwerkstoff zufällig verteilt und ausgerichtet. Die Faserverbundwerkstoffe sind in diesem Fall in allen Richtungen in gleicher Weise belastbar. In einer anderen Ausführungsform sind die metallischen Langfasern und/oder Endlosfasern im Faserverbundwerkstoff in Vorzugsrichtungen ausgerichtet, wobei die einzelnen Fasern im Wesentlichen parallel zueinander liegen. In diesem Fall ist es möglich, die Biegefestigkeit der erhaltenen Faserverbundwerkstoffe in ausgewählten Richtungen (nämlich entlang der Ausrichtung der Fasern) zu erhöhen.

Um eine authentische oder annähernd authentische Rekonstruktion der Defektstelle zu ermöglichen, stellt das Implantat ein vollständiges oder zumindest teilweise dreidimensionales Abbild der zu reparierenden Defektstelle des Knochens dar. Dadurch kann gewährleistet werden, dass das Implantat die fehlende Funktion der Knochendefektstelle teilweise oder vollständig übernehmen kann.

Textiltechnisch hergestellte Faserstrukturen aus metallischen Fasern, die aufgrund ihrer Vielfalt zur Herstellung von Implantaten beliebiger Abmessung und Form geeignet sind, eignen sich daher vorteilhaft für eine Rekonstruktion der Defektstelle des Knochens. Mit dem erfindungsgemäßen Implantat kann eine authentische Rekonstruktion der Defektstelle des Knochens somit patientenindividuell erfolgen.

Erfindungsgemäß finden gerüstgebende Faserstrukturen in zylindrischer Form beispielsweise Anwendung für die Herstellung von standardisierten Zwischenwirbelimplantaten (einzusetzen bei Wirbelrevisionen) oder als Augmentat für den Schienbeinkopf (einzusetzen bei Knierevisionen). Gerüstgebende Faserstrukturen für plattenförmige Implantate finden beispielsweise Anwendung in Revisionselementen bei Frakturen im Schulterbereich, im Beckenbereich oder bei Schädelfrakturen.

Es können ausschließlich metallische Fasern in erfindungsgemäßen Faserbundwerkstoffen eingesetzt werden, optional können auch andere Fasern, enthalten sein. Die anderen Fasern sind dabei bevorzugt Fasern aus einem oder mehreren Polymeren, vorzugsweise aus einem oder mehreren resorbierbaren Polymeren.

Als Metalle sind für erfindungsgemäße Implantate sowohl Metalle geeignet, die im Körper nicht abgebaut werden (nicht-resorbierbare Metalle) als auch Metalle, die im Körper korrodieren und dabei sukzessive abgebaut werden (resorbierbare Metalle).

Die metallischen Fasern bestehen in einer Ausgestaltung der Erfindung aus einem nichtresorbierbaren Metall, vorzugsweise ausgewählt aus Titan, Tantal, Niob, Gold, Silber, Kobalt, Rhenium, Hafnium, Legierungen enthaltend die vorgenannten Metalle (vorzugsweise zu mindestens 60 Gew.-%) und Edelstahllegierungen. Bevorzugt sind davon Titan, Titanlegierungen oder Edelstahllegierungen, besonders bevorzugt Titan oder Titanlegierungen.

In einer weiteren Ausgestaltung der Erfindung bestehen die metallischen Fasern aus einem resorbierbaren Metall (hierin auch als biokorrodierbar bezeichnet), vorzugsweise ausgewählt aus reinem Eisen, Zink, Magnesium, unedlen Eisenlegierungen, Zink-Legierungen oder Magnesiumlegierungen. Bevorzugt sind davon reines Eisen, unedle Eisenlegierungen, Magnesium oder Magnesiumlegierungen, besonders bevorzugt Magnesium oder Magnesiumlegierungen.

In einer weiteren Ausgestaltung der Erfindung werden metallische Fasern (resorbierbare Metallfasern und nicht-resorbierbare Metallfasern, wie zuvor beschrieben, sind dafür gleichermaßen geeignet) eingesetzt, die eine metallische Beschichtung (mit einem weiteren Metall, das nicht identisch zum Metall des Faserkerns ist) oder eine Beschichtung mit einem nicht-metallischen bioverträglichen Material aufweisen. Durch die Beschichtung wird vorteilhaft die Bioverträglichkeit der metallischen Fasern erhöht. Bevorzugte Metalle für die Beschichtung sind Titan, Titanlegierungen (bevorzugt Ti6Al4V, Ti5Al4Nb, Ti5Al2.5Fe oder Nitinol), Edelstahl-Legierungen, Kobalt-Basis-Legierungen sowie Tantal, Niob, Molybdän, Rhenium, Hafnium und deren Legierungen. Besonders bevorzugt davon sind Titan, Titanlegierungen, Edelstahl-Legierungen oder Kobalt-Basis-Legierungen. Bevorzugte nichtmetallische bioverträgliche Materialien für die Beschichtung sind vorzugsweise ausgewählt aus Calciumphosphaten, Magnesiumphosphaten, Magnesiumoxid, Titandioxid und/oder Titannitrid (vorzugsweise Titandioxid und/oder Titannitrid).

Um in den Faserverbundwerkstoffen eine bessere Haftung zwischen den metallischen Fasern und dem Matrixmaterial, also dem mineralischen Knochenzement, zu erzielen, ist die Oberfläche der metallischen Fasern vorzugsweise oberflächenbehandelt. Bevorzugt ist die Oberfläche der metallischen Fasern aufgeraut, silikatisiert, phosphatiert, eloxiert oder anodisiert. Dadurch wird vorteilhaft eine bessere Spannungsübertragung zwischen den Materialien des Faserverbundwerkstoffs erzielt.

Die Druckfestigkeit eines erfindungsgemäßen Implantats beträgt vorzugsweise > 50 MPa, bevorzugt > 80 MPa. Die Biegefestigkeit (4-Punkt-Biegefestigkeit) eines erfindungsgemäßen Implantats beträgt vorzugsweise > 10 MPa, vorzugsweise > 15 MPa. Die Zugfestigkeit eines erfindungsgemäßen Implantats beträgt vorzugsweise >10 MPa.

Mit den erfindungsgemäßen Implantaten, die mehrlagige und/oder mit Stehfäden verstärkte Geflechtarten enthielten wurden maximale Biegefestigkeiten von ca. 85-95 MPa erreicht.

Bevorzugt enthält der mineralische Knochenzement, insbesondere das mineralische Knochenzementpulver, Silikate, Phosphate, Sulfate, Carbonate, Oxide und/oder Hydroxide in Verbindung mit Calciumionen, Magnesiumionen und/oder Strontiumionen. Davon bevorzugt sind Calciumphosphate und/oder Magnesiumphosphate. Bevorzugt enthält der mineralische Knochenzement Calcium- und/oder Magnesiumsalze der Ortho-Phosphorsäure, der dimeren oder polymeren Phosphorsäure, Glycerophosphorsäure und weiterer mono- oder disubstituierter organischer Phosphorsäureester, besonders bevorzugt sind Calcium- und/oder Magnesiumsalze der Ortho-Phosphorsäure. Besonders bevorzugt enthält der mineralische Knochenzement mindestens eine der folgenden Verbindungen: Monocalciumphosphat Monohydrat, Monocalciumphosphat Anhydrit, Dicalciumphosphat Anhydrit, Dicalciumphosphat Dihydrat, Octacalciumphosphat, α-Tricalciumphosphat, ß-Tricalciumphosphat, amorphes Calciumphosphat, Hydroxylapatit, Calcium-defizientes Hydroxylapatit, substituiertes Hydroxylapatit, nichtstöchiometrisches Hydroxylapatit, Nano-Hydroxylapatit, Tetracalciumphosphat, Calciumsulfat, Calciumsulfat Hemihydrat, Calciumsulfat Dihydrat, Calciumoxid, Calciumhydroxid, Calciumcarbonat, Calciumglycerophosphat, Calciumcitrat, Calciumlactat, Calciumacetat, Calciumtartrat, Calciumchlorid, Calciumsilikat, Magnesiumhydrogenphosphat, Trimagnesiumphosphat, Magnesiumdihydrogenphosphat, Magnesiumchlorid, Magnesiumglycero-phosphat, Magnesiumhydroxid, Magnesiumhydroxidcarbonat, Magnesiumoxid (MgO), Magnesiumcitrat, Calcium-Magnesiumcarbonat (Dolomit), Magnesiumsilikate.

Das mineralische Knochenzementpulver kann ausschließlich aus mineralischen Komponenten bestehen. Optional enthält das mineralische Knochenzementpulver resorbierbare polymere Zusätze, vorzugsweise ausgewählt aus Chitosan, Hyaluronsäure, Gelatine, Chondroitinsulfat, Cellulosederivate, Stärkederivate, Alginat, wasserlösliche Acrylate, Polyethylenglycol, Polyethylenoxid, Polyvinylpyrrolidon und Copolymeren aus wasserlöslichen Acrylaten mit Polyethylenglycol und/oder Polyethylenoxid.

In einer Ausgestaltung der Erfindung liegt das mineralische Knochenzementpulver vor der Auslösung der Aushärtereaktion als pulverförmiger Feststoff vor. Zur Auslösung der Aushärtereaktion wird das mineralische Knochenzementpulver mit Wasser oder einer wässrigen Lösung zu einer pastösen Zubereitung vermengt, mit der zur Herstellung des erfindungsgemäßen Implantats die metallischen Langfasern und/oder Endlosfasern, die bevorzugt in Form eines Faserhalbzeugs vorliegen, imprägniert werden.

Ein erfindungsgemäßes Implantat ist bevorzugt erhältlich durch die Imprägnierung eines Faserhalbzeugs, welches metallische Langfasern und/oder metallische Endlosfasern enthält oder daraus besteht, mit einer pastösen Zubereitung, welche resorbierbares mineralisches Knochenzementpulver dispergiert in einer Flüssigkeit enthält.

In einer bevorzugten Ausgestaltung der Erfindung liegt das mineralische Knochenzementpulver vor der Auslösung der Aushärtereaktion in Form einer wasserfreien Zubereitung vor, die das mineralische Knochenzementpulver dispergiert in einer nicht-wässrigen und wasserfreien Trägerflüssigkeit enthält. Die wasserfreie Zubereitung liegt in pastöser Form vor. Zur Herstellung des erfindungsgemäßen Implantats werden die metallischen Langfasern und/oder Endlosfasern, die bevorzugt in Form eines Faserhalbzeugs vorliegen, mit der wasserfreien Zubereitung imprägniert (bevorzugte Variante). Alternativ dazu werden die metallischen Langfasern und/oder Endlosfasern, die bevorzugt in Form eines Faserhalbzeugs vorliegen, zur Herstellung des erfindungsgemäßen Implantats mit der wasserfreien Zubereitung imprägniert, die zuvor mit Wasser oder einer wässrigen Lösung kontaktiert oder vermischt wurde.

Geeignete wasserfreie pastöse Zubereitungen weisen im Sinne der Erfindung bei Raumtemperatur bevorzugt eine Viskosität zwischen 0,1 und 350.000 mPa·s und besonders nicht höher als 200.000 mPa·s auf.

Die Viskosität der Zementpasten kann bei gleichem Pulver/Flüssigkeits-Verhältnis durch die Einstellung der Partikelgrößenverteilung beeinflusst werden. Zementpulver mit groben Partikeln haben eine geringere Viskosität nach dem Anmischen, zeigen jedoch auch geringere Festigkeiten nach Aushärtung. Daher werden die Zemente so aufbereitet, dass die Eigenschaften auf eine gewünschte Viskosität angepasst werden. Ganz besonders vorteilhaft sind Zementpulver mit einer breiten Partikelgrößenverteilung, bei der ein Bereich von <1µm bis >50µm abgedeckt wird. Besonders bevorzugt sind Pulverzubereitungen bei denen >20% der Pulvermasse eine Partikelgröße von <5µm, >20% eine Partikelgröße von 10-50µm und >10% eine Partikelgröße von >50µm aufweisen.

Durch die Einstellung der Partikelgrößen des mineralischen Knochenzementpulvers in einem mittleren Bereich von ca. 8µm, einer Partikelgrößenverteilung von <1µm bis >50µm und des Pulver-Flüssigkeitsverhältnisses bei der Zementanmischung auf I/p 0,5 werden vorteilhaft Zemente erhalten, die eine gute Befüllung dichter Drahtstrukturen ermöglichen und gute mechanische Festigkeiten liefern. Zudem weisen die erhaltenen Zementmischungen Druckfestigkeiten von bis zu 100 MPa auf.

In einer bevorzugten Ausgestaltung der Erfindung umfasst das erfindungsgemäße Implantat metallische Langfasern und/oder metallische Endlosfasern in Kombination mit einer wasserfreien Zubereitung in Form einer Paste, in der mineralisches Knochenzementpulver in einer wasserfreien und nicht-wässrigen, vorzugsweise einer organischen, Trägerflüssigkeit dispergiert ist. Ohne Kontakt mit Wasser reagiert das mineralische Zementpulver bei dieser Anwendung nicht zu einem schwerlöslichen Festkörper, so dass das Implantat in dieser Form flexibel verformbar und leicht zu handhaben ist und in einfacher Weise an die zu behandelnde Knochendefektstelle angepasst werden kann. Dabei ist es vorteilhaft möglich, das Implantat direkt ohne vorherige Aushärtung des als pastöse Zubereitung vorliegenden mineralischen Knochenzements zur Implantation einzusetzen. In diesem Fall härtet der mineralische Knochenzement im Körper durch Kontakt mit der Körperflüssigkeit zu dem schwerlöslichen Festkörper aus. Durch die Verstärkung mit den metallischen Fasern ist jedoch bereits zum Zeitpunkt der Implantation eine hinreichende Stützwirkung gegeben. Alternativ besteht die Möglichkeit, das mineralische Knochenzementpulver durch Kontakt mit Wasser oder einer wässrigen Lösung bereits vor der Implantation zur Aushärtung zu bringen.

Unter wasserfrei im Sinne der Erfindung wird ein Gewichtsanteil von weniger als 1 Gew.-% Wasser, bevorzugt weniger als 0,1 Gew.-%, Wasser verstanden. Die Trägerflüssigkeit ist bevorzugt eine organische Trägerflüssigkeit, in der das mineralische Knochenzementpulver dispergiert ist. Die organische Trägerflüssigkeit ist so ausgewählt, dass sie selbst nicht mit dem mineralischen Knochenzementpulver reagiert. Grundsätzlich sind sowohl wasserlösliche als auch schwer wasserlösliche Trägerflüssigkeiten geeignet. Unter schwer wasserlöslich werden im Sinne der Erfindung Verbindungen verstanden, deren maximale Löslichkeit in Wasser 1,0 mol/l, bevorzugt 0,1 mol/l, beträgt. Verbindungen mit einer maximalen Löslichkeit in Wasser von mehr als 1 mol/l (vorzugsweise mehr als 3 mol/l) werden hierin als wasserlöslich bezeichnet. Bevorzugt sind jedoch schwer wasserlösliche Trägerflüssigkeiten. Besonders bevorzugt sind hydrophobe Trägerflüssigkeiten. Die in der wasserfreien Zubereitung enthaltene Trägerflüssigkeit ist vorzugsweise bioverträglich. Der Gewichtsanteil der Trägerflüssigkeit bezogen auf die Gesamtmasse der wasserfreien Zubereitung beträgt bevorzugt 5 bis 25 Gew.-%, vorzugsweise 5 bis 20 Gew.-%, vorzugsweise 5 bis 15 Gew.-%, vorzugsweise 5 bis 12,5 Gew.-%.

Bevorzugte schwer wasserlösliche Trägerflüssigkeiten, die in der wasserfreien Zubereitung enthalten sind, sind ausgewählt aus Glycerintriacetat, Glycerintributyrat, Glycerintrioleat, Glycerindioleat, Glycerinmonooleat, Caprylcaprat, Decyloleat, Isopropylmyristat, Isopropylpalmitat, Ölsäure, Oleylalkohol, Oleyloleat, kurzkettigen Triglyceriden, mittelkettigen Triglyceriden, kurz- und mittelkettigen Fettsäureestern des Propylenglycols, Ethylbenzoylacetat, Ethylbutyrat, Ethylbutyrylacetat, Ethyloleat, Ethylcaproat, Ethylcaprylat, Ethylcaprat, Ethyllaurat, Ethyllävulinat, Ethylmyristat, Ethylpalmitat, Ethyllinoleat, Ethylstearat, Ricinolsäure, Linolsäure, Linolensäure, Arachinsäure, Oleinsäure, Ethylarachidat, α-Tocopherol, ß-Tocopherol, γ-Tocopherol, δ-Tocopherol, Benzylalkohol, Benzylbenzoat, Diethylbutylmalonat, Diethylenglycoldibutylether, Diethylethylmalonat, Diethylphenylmalonat, Diethylphthalat, Diethylsebaceat, Diethylsuberat, Diethylsuccinat, Dibutylmaleinat, Dibutylphthalat, Lecithin, Paraffinöl, Petrolatum, flüssigen Paraffinen und Estern der Sebacinsäure. Besonders bevorzugte Trägerflüssigkeiten sind kurz- oder mittelkettige Triglyceride oder mittelkettige Fettsäureester des Ethylenglycols und des Propylenglycols. Unter kurzkettigen Fettsäureverbindungen werden Verbindungen von Fettsäuren einer Länge von jeweils 2 bis 5 Kohlenstoffatomen verstanden. Unter mittelkettigen Fettsäureverbindungen werden Verbindungen von Fettsäuren einer Länge von jeweils 6 bis 14 Kohlenstoffatomen verstanden. Besonders bevorzugte schwer wasserlösliche Trägerflüssigkeiten, die in der wasserfreien pastösen Zubereitung enthalten sind, sind ausgewählt aus Estern aus Fettsäuren und ein- oder mehrwertigen Alkoholen. Davon bevorzugt sind Triglyceride. Ganz besonders bevorzugt davon sind Ester aus Triglyceriden mit Fettsäuren, die durchschnittlich weniger als 14 C-Atome aufweisen. Weiter bevorzugte schwer wasserlösliche Trägerflüssigkeiten sind Polypropylenglykole und Ester von Polypropylenglykolen sowie Mono- und Di-Ether von Polypropylenglykolen mit Monoalkoholen.

Bevorzugte wasserlösliche Trägerflüssigkeiten, die in der wasserfreien Zubereitung enthalten sind, sind ausgewählt aus Polymeren des Ethylenglykols, kurzkettigen Oligomeren des Propylenglykols, Co-Polymeren mit Ethylenglykol und Propylenglykoleinheiten, Mono- und Di-Methylether des Polyethylenglycols, Glycerin und dessen wasserlöslichen Ethern und Estern und Di- und Polyglycerin.

Weitere Bestandteile der wasserfreien Zubereitung sind vorzugsweise Tenside, bevorzugt mindestens ein nichtionisches Tensid, besonders bevorzugt mindestens ein anionisches und mindestens ein nichtionisches Tensid. Das Gewichtsverhältnis der insgesamt in der wasserfreien pastösen Zubereitung enthaltenen Feststoffe zur Summe des Gewichts der organischen Trägerflüssigkeit und der Tenside beträgt dabei bevorzugt mehr als 5, vorzugsweise mehr als 6, besonders bevorzugt mehr als 10. Dies hat den Vorteil, dass die mineralische Knochenzementmatrix nach der Aushärtung eine besonders hohe Druckfestigkeit aufweist.

Ein weiterer Bestandteil des mineralischen Knochenzements, vorzugsweise der wasserfreien Zubereitung, ist vorzugsweise mindestens ein Abbindebeschleuniger. Dadurch werden vorteilhaft die Abbindezeit und der pH-Wert Verlauf bei der Aushärtung der erfindungsgemäßen Zubereitung eingestellt. Bevorzugte Abbindebeschleuniger sind Phosphatsalze, organische Säuren oder Salze organischer Säuren. Bevorzugt sind Natrium- und/oder Kaliumionenhaltige Phosphate oder Natrium- und/oder Kaliumionenhaltige Salze organischer Säuren. Besonders bevorzugt sind kaliumionenhaltige Phosphate (vorzugsweise Kaliumphosphate, insbesondere Kaliumdihydrogenphosphat und di-Kaliumhydrogenphosphat). Mit kaliumionenhaltigen Phosphaten wird eine besonders vorteilhafte Abbindekinetik, besonders in Kombination mit nichtionischen Tensiden (ganz besonders gut in Kombination mit nichtionischen und anionischen Tensiden), erzielt werden. Der Abbindebeschleuniger ist bevorzugt in einem Massenanteil (in Bezug auf die Masse des mineralischen Zementpulvers) von 0,1 bis 5 %, besonders bevorzugt 0,2 bis 4 %, ganz besonders bevorzugt 0, 5 - 3, 5 % in der wasserfreien pastösen Zubereitung enthalten.

In einer Ausgestaltung der Erfindung enthält die wasserfreie Zubereitung Tenside und/oder einen Abbindebeschleuniger und/oder wasserlösliche Füllstoffe.

Weitere bevorzugte Bestandteile des mineralischen Knochenzements sind Wirkstoffe. Bevorzugt sind pharmazeutische Wirkstoffe mit einer wachstumsstimulierenden oder antimikrobiellen Wirkung. Besonders bevorzugte Wirkstoffe sind ausgewählt aus Antibiotika, Antiseptika, antimikrobiellen Peptiden, antiresorptive Wirkstoffe (vorzugsweise Bisphosphonate, Cortikoide, Fluoride, Protonenpumpen-Inhibitoren), knochenwachstumsstimulierende Wirkstoffe (vorzugsweise Wachstumsfaktoren, Vitamine, Hormone, Morphogene, davon bevorzugt knochenmorphogenetische Proteine). Das erfindungsgemäße Implantat eignet sich besonders gut als Wirkstoffträger. Einerseits liegt dies daran, dass die Bereitstellung des erfindungsgemäßen Implantats bei niedrigen Temperaturen (vorzugsweise unter 50 °C) gearbeitet wird. Dies erlaubt die problemlose Einarbeitung von temperatursensitiven Wirkstoffen. Andererseits bietet das erfindungsgemäße Implantat eine verbesserte Wirkstofffreisetzung, da die Wirkstoffe durch die Resorption des mineralischen Knochenzements sukzessive freigesetzt werden. Damit erlaubt das erfindungsgemäße Implantat eine kontrollierte Wirkstofffreisetzung und ist für den Einsatz temperatursensitive Wirkstoffe in besonders vorteilhafter Weise geeignet.

Weitere bevorzugte Bestandteile des mineralischen Knochenzements, vorzugsweise der wasserfreien Zubereitung, sind Füllstoffe. Bevorzugt sind wasserlösliche partikuläre Füllstoffe aus mineralischen oder organischen Substanzen. Durch den Einsatz wasserlöslicher Partikel kann vorteilhaft die Porosität des bei Aushärtung mit Wasser gebildeten Feststoffs eingestellt werden. Wasserlösliche Füllstoffe weisen vorzugsweise eine Partikelgröße von 50 µm bis 2000 µm, bevorzugt von 100 µm bis 1000 µm, auf. Wasserlösliche Füllstoffe sind in der wasserfreien Zubereitung bevorzugt zu 5 bis 90 Vol.-%, vorzugsweise 10 bis 80 Vol.-% enthalten (bezogen auf das Gesamtvolumen der wasserfreien Zubereitung). Bevorzugte wasserlösliche Füllstoffe sind ausgewählt aus Zuckern (vorzugsweise Saccharose), Zuckeralkoholen (vorzugsweise Sorbit, Xylit, Mannit), wasserlöslichen Salzen (vorzugsweise Natriumchlorid, Natriumcarbonat oder Calciumchlorid). Bevorzugt werden die wasserlöslichen Füllstoffe in Form von Granulaten eingesetzt. Der Porenanteil des ausgehärteten mineralischen Knochenzements beträgt bevorzugt 10 bis 75 Vol.-%, besonders bevorzugt 10 bis 50 Vol.-%. Die Poren weisen bevorzugt eine mittlere Porenweite (maximale innere Ausdehnung einer Pore) von 100 - 2500 µm, besonders bevorzugt 100 - 1000 µm auf.

Besonders bevorzugt sind sämtliche Bestandteile des mineralischen Knochenzements im Körper resorbierbar. Besonders bevorzugt für die Erfindung ist die Kombination von resorbierbaren metallischen Fasern und dem resorbierbaren mineralischen Knochenzement.

In einem erfindungsgemäßen Implantat beträgt der Gewichtsanteil der metallischen Fasern am gesamten Faserverbundwerkstoff vorzugsweise 1 - 90 Gew.-%, bevorzugt 5-75 Gew.-%, besonders bevorzugt 10 - 75 Gew.-%, ganz besonders bevorzugt 25 - 75 Gew.-%.

Die Erfindung umfasst auch ein Verfahren zur Herstellung eines erfindungsgemäßen Implantats, vorzugsweise eines Knochenimplantats, welches ein derartiges Implantat enthält oder daraus besteht. Das Verfahren umfasst die Bereitstellung des Faserverbundwerkstoffs mit den Schritten:
- Bereitstellung einer pastösen Zubereitung, welche resorbierbares mineralisches Knochenzementpulver dispergiert in einer Flüssigkeit, vorzugsweise Wasser, einer wässrigen Lösung oder einer nicht-wässrigen und wasserfreien Trägerflüssigkeit, enthält, und
- Imprägnierung mehrerer metallischer Langfasern und/oder metallischer Endlosfasern mit einem Aspektverhältnis von mindestens 100 : 1, in Form gerüstgebender Faserstrukturen mit der pastösen Zubereitung; vorzugsweise liegen die metallischen Fasern in Form eines, vorzugsweise vollständig metallischen, Faserhalbzeugs vor, welches die metallischen Langfasern oder metallischen Endlosfasern enthält.

Im ersten Schritt des erfindungsgemäßen Verfahrens wird eine pastöse Zubereitung mit mineralischem Knochenzementpulver bereitgestellt. Dafür wird in einer Ausgestaltung des Verfahrens mineralisches Knochenzementpulver in Wasser oder einer wässrigen Lösung dispergiert, so dass eine aushärtende Knochenzementzubereitung in pastöser Form erhältlich ist. Mit dieser Zubereitung werden anschließend die metallischen Fasern, vorzugsweise das Faserhalbzeug, imprägniert.

In einer anderen Ausgestaltung des Verfahrens wird mineralisches Knochenzementpulver in einer wasserfreien Trägerflüssigkeit dispergiert, so dass eine wasserfreie Zubereitung in pastöser Form erhältlich ist. Die wasserfreie Zubereitung wird in einer Ausführungsvariante mit Wasser oder einer wässrigen Lösung kontaktiert oder vermischt, wodurch die Aushärtereaktion des Knochenzements ausgelöst wird. Anschließend werden die metallischen Fasern, vorzugsweise das Faserhalbzeug, mit dieser, aushärtenden aber noch pastösen, Zubereitung imprägniert. In einer anderen, besonders bevorzugten Ausführungsvariante werden die metallischen Fasern, vorzugsweise das Faserhalbzeug, mit der wasserfreien Zubereitung imprägniert, ohne dass diese zuvor mit Wasser oder einer wässrigen Lösung in Kontakt gebracht wurde. Das Resultat dieses zuletzt genannten Verfahrens ist ein formbares Implantat aus einem flexiblen Faserverbundwerkstoff, der direkt implantiert werden kann und allein durch die Anwesenheit der Körperflüssigkeit sukzessive zu einem Festkörper aushärtet.

Die Imprägnierung des Faserhalbzeugs mit der pastösen Zubereitung wird vorzugsweise unter erhöhtem Druck, vorzugsweise im Bereich von mehr als 1 bis 100 MPa, durchgeführt. Dies hat den Vorteil, dass eine nahezu porenfreie Knochenzementmatrix erhalten werden kann und dadurch besonders hohe Druckfestigkeiten erzielt werden können. Dies wird bevorzugt bewerkstelligt, indem das Faserhalbzeug in eine Form eingebracht wird und die pastöse Zubereitung zunächst ohne zusätzliche Druckbeaufschlagung in das Porensystem des metallischen Halbzeugs eingefüllt wird (vorzugsweise wird dies durch Rütteln oder Vibration unterstützt). Anschließend wird die Form verschlossen und mit einem Druck von bis zu 100 MPa beaufschlagt. Auf diese Weise kann das Volumen der im Knochenzement verbleibenden Luftblasen auf 1/1000 (im Vergleich zur Befüllung des Faserhalbzeugs ohne Druckbeaufschlagung) reduziert werden.

Nach einer vorteilhaften Ausgestaltung des erfindungsgemäßen Verfahrens wird das Faserhalbzeug in eine Schalungsform eingebracht und eine pastöse Zubereitung in das Porensystem des metallischen Halbzeugs unter mechanischer Bewegung eingefüllt, wobei die gerüstgebende Faserstruktur von der pastösen Masse umschlossen wird. Vorzugsweise wird der beschriebene Vorgang durch Rütteln oder Vibration unterstützt, wobei gleichzeitig Gase (z.B. Luft oder Inertgase) aus dem Formkörper ausgetrieben werden.

Bei einer wasserhaltigen pastösen Zubereitung härtet die pastöse Zubereitung nach dem Kontaktieren mit den gerüstgebenden Faserstrukturen aus. In Abhängigkeit der gewünschten Form und Oberflächenstruktur des erfindungsgemäßen Implantats, kann dieses vor dem Aushärten formgebend verpresst werden.

Gerade bei dichteren, mehrlagigen gerüstgebenden Faserstrukturen (z.B. 3x- bzw. 4x-Geflecht) ist eine vollständige Benetzung aller Drähte durch Beaufschlagung mit Druck zu gewährleisten. Nach Applikation und Verteilung des Zementes in der ganzen Probe kann eine Zementmischung, die dünnflüssig genug ist, noch gut zwischen die maschigen bzw. porigen gerüstgebenden Faserstrukturen hineinfließen.

Auch für den Fall, dass die Aushärtung des mineralischen Knochenzements vor der Implantation stattfindet, wird zur Aushärtung keine keramische Sinterung durchgeführt. Die Aushärtung erfolgt daher vorzugsweise bei einer Temperatur unterhalb von 250 °C, besonders bevorzugt unterhalb von 100 °C und weiter bevorzugt unterhalb von 50°C.

Des Weiteren kann eine befüllte gerüstgebende Faserstruktur vor der Aushärtung durch äußere Formgebungsprozesse in eine definierte dreidimensionale Form gebracht werden, z.B. eine zylindrischen Struktur in eine Platte gepresst werden.

Das erfindungsgemäße Implantat kann bereits allein als Knochenimplantat eingesetzt werden, oder in Kombination mit weiteren in Knochenimplantaten üblichen Elementen zu einem komplexen Knochenimplantat zusammengesetzt sein. In einer bevorzugten Ausgestaltung besteht das Knochenimplantat bevorzugt nur zu einem Teil aus dem erfindungsgemäßen Implantat. Neben dem erfindungsgemäßen Implantat können dabei beliebige andere, vorzugsweise metallische, Formteile im erfindungsgemäßen Knochenimplantat enthalten sein, die fest mit dem Implantat verbunden sind. Entsprechende Formteile und Verbindungsmöglichkeiten sind aus dem Stand der Technik bekannt. In einem bevorzugten Fall besteht das Knochenimplantat aus einer (vorzugsweise metallischen) Platte oder einem (vorzugsweise metallischen) Nagel, wobei die Platte oder der Nagel jeweils über metallische Schrauben am Knochen befestigt wird. Ebenfalls bevorzugt sind Knochen-Gelenk-Implantate, wobei die dem Knochen zugewandte Seite aus dem erfindungsgemäßen Implantat besteht oder ein erfindungsgemäßes Implantat enthält und die dem Gelenk zugewandte Seite aus einem soliden Metall oder einer soliden Keramik besteht. Beispiele dafür sind kombinierte Hüftpfannen, die Komponenten von Knieprothesen und der Oberflächenersatz von Hüftköpfen.

Daher ist von der Erfindung auch ein Knochenimplantat umfasst, welches aus mindestens einem erfindungsgemäßen Implantat besteht oder welches mindestens einen erfindungsgemäßes Implantat in Kombination mit einem Bauteil aus solidem Metall oder aus einer soliden Keramik enthält.

Die Erfindung umfasst auch ein Set zur Herstellung eines erfindungsgemäßen Implantats, vorzugsweise eines Knochenimplantats, welches ein derartiges Implantat enthält oder daraus besteht. Das Set umfasst die folgenden Komponenten:
a) mindestens eine Knochenzementzubereitung, umfassend resorbierbares mineralisches Knochenzementpulver,
b) mehrere metallische Langfasern und/oder Endlosfasern, in Form einer gerüstgebenden Faserstruktur (bevorzugt vollständig metallischen) vorzugsweise Faserhalbzeug.

Die Knochenzementzubereitung eines erfindungsgemäßen Sets liegt bevorzugt wie folgt konfektioniert vor:
- Einkomponentensystem:
   o mineralisches Knochenzementpulver oder
   o mineralisches Knochenzementpulver dispergiert in einer, vorzugsweise organischen, Trägerflüssigkeit (wasserfreie Zubereitung); oder
- Zweikomponentensystem mit jeweils separat verpackten Komponenten:
   o mineralisches Knochenzementpulver und Wasser oder eine wässrige Lösung,
   o mineralisches Knochenzementpulver dispergiert in einer vorzugsweise organischen Trägerflüssigkeit (wasserfreie Zubereitung) und Wasser oder eine wässrige Lösung (wasserhaltige Zubereitung); optional sind in der wässrigen Lösung nicht mit Wasser zu einem Feststoff reagierende partikuläre Feststoffe dispergiert, so dass die wässrige Lösung die Form einer Paste annimmt.

Besonders bevorzugt liegt die Knochenzementzubereitung in einem erfindungsgemäßen Set als wasserfreie Zubereitung (Einkomponentensystem, ganz besonders bevorzugte Variante) oder als wasserfreie Zubereitung in Kombination mit einer wasserhaltigen Zubereitung (Zweikomponentensystem) vor. Besonders bevorzugt ist in diesem Fall zumindest die Knochenzementzubereitung in eine wasserdichte Verpackung eingebracht.

Ferner umfasst die Erfindung die Verwendung eines erfindungsgemäßen Implantats oder eines erfindungsgemäßen Knochenimplantats zur Behandlung eines Knochendefekts. Ein bevorzugtes Anwendungsgebiet ist die Osteosynthese. In diesem Gebiet ist eine hohe Bioaktivität des Implantats erwünscht, eine Materialentfernung nach einer erfolgten Frakturheilung jedoch zu vermeiden. Die Erfindung umfasst auch die Verwendung eines erfindungsgemäßen Sets zur Herstellung eines Knochenimplantats.

Anhand folgender Figuren und Ausführungsbeispiele soll die Erfindung näher erläutert werden, ohne die Erfindung auf diese zu beschränken.
- **Fig. 1**: Kraft-Weg-Diagramm, Herausziehen eines Titandrahtes aus ausgehärtetem Magnesium-Calciumphosphat-Zement
- **Fig. 2**: Kraft-Weg-Diagramm 4-Punkt-Biegung, erfindungsgemäßes Implantat (Reintitanfasergelege und Magnesium-Calciumphosphat-Zement) und Referenzprobe (unverstärkter Magnesium-Calciumphosphat-Zement)
- **Fig. 3**: Deformationskurven von Referenzmaterial und 2x-Geflecht+SF-verstärkten Platten bei Biegung
- **Fig. 4:**: (A) zeigt ein unbehandeltes Titangeflecht, welches im Verbund mit einem Magnesium-Calciumphosphat-Zement ein erfindungsgemäßes Faserverbundmaterial (B) darstellt, wobei das metallhaltige Geflecht in den Magnesium-Calciumphosphat-Zement inkludiert ist.
- **Fig. 5:**: Schematische Darstellung eines Augmentats für den Schienbeinkopf für die Knierevision.

### Beispiel 1: Verbundfestigkeit eines Verbundwerkstoffes eines Magnesium-Calciumphosphat-Zements mit einem Titandraht

Magnesium-Calciumphosphat-Zement der Zusammensetzung Mg_{2,5}Ca_{0,5}(PO₄)₂ wird durch Sinterung (5h bei 1050 °C) und anschließender Vermahlung von Calcium- und Magnesiumphosphaten und -Carbonaten im molaren Verhältnis der Endzusammensetzung gewonnen. Folgende Bestandteile wurden eingesetzt: 0,33 mol CaHPO₄, 0,17 mol CaCO₃, 1,67 mol MgHPO₄ * 3 H₂O und 0,83 mol Mg(OH)₂. Das so erhaltene Knochenzementpulver wurde mit einer wässrigen 3,5 mol/l Ammoniumphosphatlösung im Verhältnis 0,5 (Masse Flüssigkeit zu Masse Knochenzementpulver) angemischt. In die aushärtende Masse wurde ein Titandraht der Legierung Ti6Al4V mit einem Durchmesser von 0,5 mm auf einer Länge von 5 mm einzementiert. Nach 72-stündiger Inkubation unter physiologischer Kochsalzlösung bei 37°C wurde der Draht mit einer Universalprüfmaschine aus dem Zementkörper gezogen.

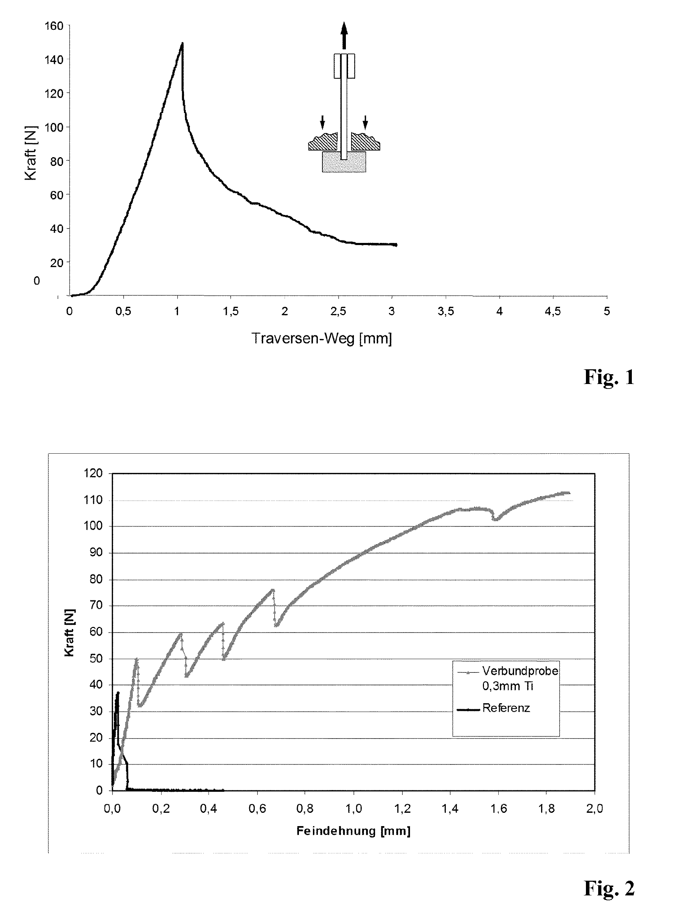

Fig. 1 zeigt das Kraft-Weg-Diagramm beim Herausziehen des Drahts aus der Zementmatrix. Der Kraftverlauf zeigte zunächst eine Phase mit hoher Lastaufnahme bis zu einem maximalen Wert von bis zu 150 N. Bei Überschreitung der maximalen Last verblieb über einen weiteren Auszugsweg von 2 mm eine Restkraft von ca. 40 N bis 20 N erhalten, was zeigt, dass auch bei einer Lastüberschreitung kein katastrophales Versagen eines erfindungsgemäßen Verbundmaterials zu erwarten ist, sondern eine beachtliche Bruchresistenz besteht. Es ist zu erwarten, dass dieses Verhalten umso ausgeprägter ist, je mehr Fasern parallel zur Belastungsrichtung vorliegen. Der absolute Wert für die Auszugskraft des Drahts aus der Zementmatrix zeigt eine unerwartet hohe Haftfestigkeit vom Titandraht in der Zementmatrix, sowohl in der Haft-, als auch in der Gleitreibung.

### Beispiel 2: Biegefestigkeit eines erfindungsgemäßen Implantats mit Magnesium-Calciumphosphat-Zement und einem Geflecht aus Edelstahldrähten in Form eines Faserverbundwerkstoffs

Ein Geflecht aus Endlosfasern aus Edelstahl (Faserdurchmesser 0,2 mm) wurde mit Magnesium-Calciumphosphat-Zement der Zusammensetzung gemäß Beispiel 1 imprägniert. Der Gewichtsanteil des metallischen Geflechts am Faserverbundwerkstoff betrug 3 Gew.-%.

Bereits bei diesem geringen Anteil wurde eine Erhöhung der Zugfestigkeit auf 320% und eine Erhöhung der Biegefestigkeit auf 180% erreicht. Die besten Werte wurden bei den mit Stehfäden verstärkten Varianten erreicht.

| | Zugfestigkeit [MPa] | Biegefestigkeit [MPa] |
|---|---|---|
| Magnesium-Calciumphosphat-Zement | 3,13 | 5,9 |
| Implantat mit Magnesium-Calciumphosphat-Zement und Edelstahlgeflecht | 10,1 | 10,8 |
| Implantat mit Magnesium-Calciumphosphat-Zement und 1x-Edelstahlgeflecht mit Stehfäden | 13,5 | 8,37 |

Die Zugfestigkeit wurde gemäß DIN EN ISO 527 Teil 1 und 5 bestimmt. Die 4-Punkt-Biegefestigkeit erfolgte gemäß DIN EN ISO 14125.

### Beispiel 3: Mechanische Eigenschaften eines erfindungsgemäßen Implantats mit Calciumphosphat-Zement und einzelnen Faserfilamenten aus Edelstahl in Form eines Faserverbundwerkstoffs, bei Aushärtung unter Normaldruck

Calciumphosphat-Zement der Zusammensetzung 60 Gew.-% a-Tricalciumphosphat, 26 Gew.-% Dicalciumphosphat Anhydrit, 10 Gew.-/ CaCO₃ und 4 Gew.-% Hydroxylapatit wurde mit einer 1 Gew.-%igen wässrigen Na₂HPO₄-Lösung im Verhältnis 0,4 ml Lösung je Gramm Zement in Form einer wasserfreien Zubereitung verknetet, wobei 30 Gew.-% (in Bezug auf die Gesamtmasse der Zubereitung) Edelstahlfasern (90 µm Durchmesser, 100 mm Länge) beigemischt wurden. Die so erhaltene Masse wurde in einer Silikonform der Abmessung 6x6x100 mm³ zur Aushärtung gebracht. Nach der Entformung wurden die ausgehärteten Formkörper für 100 h bei 37 °C in simulierter Körperflüssigkeit inkubiert und anschließend einer Testung unterzogen.

Die Druckfestigkeit der Formkörper wird durch die Faserverstärkung auf 140 % erhöht.

| | Druckfestigkeit [MPa] |
|---|---|
| Calciumphosphat-Zement | 17,7 |
| Implantat mit Calciumphosphat-Zement und Edelstahlfasern | 24,9 |

Die Druckfestigkeit wurde gemäß DIN EN ISO 5833 ermittelt.

### Beispiel 4: Mechanische Eigenschaften eines erfindungsgemäßen Implantats mit Calciumphosphat-Zement und einzelnen Faserfilamenten aus Edelstahl in Form eines Faserverbundwerkstoffs, bei Aushärtung unter einem Druck von 100 MPa

Edelstahlfasern und Calciumphosphat-Zement wurden analog zu Beispiel 3 zu einer pastösen Masse vermengt und in Silikonformen eingebracht. Anschließend erfolgte eine Druckbeaufschlagung mit Hilfe eines Stempels, so dass ein Druck von 100 MPa eingestellt wurde. Nach einer Stunde wurden die ausgehärteten Formkörper entformt und für 100 h bei 37 °C in simulierter Körperflüssigkeit inkubiert und anschließend einer Testung unterzogen.

Die Biegefestigkeit des erhaltenen Formkörpers war gegenüber dem Formkörper, der bei Normaldruck ausgehärtet wurde, signifikant erhöht. Es wurde ein massiver Anstieg der Druckfestigkeit auf 200 MPa im erhaltenen Formkörper festgestellt.

### Beispiel 5: Mechanische Eigenschaften eines erfindungsgemäßen Implantats mit Calciumphosphat-Zement und einzelnen Faserfilamenten aus Edelstahl in Form eines Faserverbundwerkstoffs, bei Bereitstellung des Knochenzements als wasserfreie Zubereitung

Es wurde eine wasserfreie Zubereitung aus 84 Gew.-% des Calciumphosphat-Zements, wie in Beispiel 3 beschrieben, und 16 Gew.-% einer Mischung aus Miglyol 812 (ein gesättigtes teilsynthetisches mittelkettiges Triglycerid) und Tween 80 (97 Gew.-% Miglyol und 3 Gew.-% Tween 80) hergestellt.

In die Masse wurden Edelstahlfasern, wie in Beispiel 3 beschrieben, eingemengt. Der Gewichtsanteil der Metallfasern an der Zubereitung betrug 30 Gew.-%.

Es wurde ein Strang von 8 mm Durchmesser hergestellt, in dem die Edelstahlfasern weitgehend parallel entlang des Strangs angeordnet waren. Der Strang wurde in eine längliche Form von 6x6 mm Querschnitt gepresst und anschließend durch Einlegen in Wasser zur Aushärtung gebracht. Nach der Aushärtung erfolgte eine Inkubation in simulierter Körperflüssigkeit bei 37°C für 100 h.

Anschließend wurde das Material einer mechanischen Testung unterzogen:

Die mechanische Testung ergibt für das verstärkte Material eine signifikant höhere Biegefestigkeit als für das unverstärkte Material.

### Beispiel 6: Mechanische Eigenschaften eines erfindungsgemäßen Implantats mit Magnesium-Calciumphosphat-Zement und einem zylinderförmigen Geflecht aus Edelstahlfasern in Form eines Faserverbundwerkstoffs

Magnesium-Calciumphosphat-Zement wird wie in Beispiel 1 beschrieben bereitgestellt und mit wässriger Ammoniumphosphatlösung angemischt. Die aushärtende Zementpaste wurde in einen Zylinder eingebracht, dessen Wand aus einem Geflecht von Edelstahlfasern (Durchmesser einer Faser 0,2 mm) gebildet wurde. Der Zylinder wies einen Durchmesser von 10 mm und eine Länge von 100 mm auf.

Der Zylinder wurde mit der Zementpaste vollständig gefüllt, bis die Zementpaste aus den Poren des Edelstahlgeflechts austrat. Der so erhaltene Faserverbundwerkstoff wurde bei 37 °C in simulierter Körperflüssigkeit für 100 h zur Aushärtung gebracht.

Das erfindungsgemäße Implantat wies eine 4-Punkt-Biegefestigkeit von 50 MPa auf. Ein Formkörper gleicher Abmessung aus unverstärktem Magnesium-Calciumphosphat-Zement wies eine Biegefestigkeit von <15MPa auf. Der ungefüllte Zylinder des Metallgeflechts hatte keine Biegefestigkeit. Die 4-Punkt-Biegefestigkeit wurde gemäß DIN EN ISO 5833 bestimmt.

### Beispiel 7: Mechanische Eigenschaften eines erfindungsgemäßen Implantats mit Magnesium-Calciumphosphat-Zement und einem Gelege aus Titandraht in Form eines Faserverbundwerkstoffs

Magnesium-Calciumphosphat-Zement wird wie in Beispiel 1 beschrieben bereitgestellt und mit wässriger Ammoniumphosphatlösung angemischt. Die aushärtende Zementpaste wurde in ein Gelege aus Reintitanfasern (Faserdurchmesser 0,3 mm) eingebracht. Daraus wurden Formkörper der Abmessung 80x10x6mm³ hergestellt. Der Anteil des Metalls in den Formkörpern betrug 1 Vol.-%. (entspricht einem Gewichtsanteil von 2 Gew.-%). Die Formkörper wurden für 72 h in physiologischer Kochsalzlösung bei 37 °C zur Aushärtung gebracht. Die ausgehärteten Formkörper wurden auf einer Universal-Prüfmaschine auf 4-Punkt-Biegefestigkeit untersucht.

Im Vergleich zu einem unverstärkten Formkörper derselben Dimensionen aus Magnesium-Calciumphosphat-Zement wurde bei dem erfindungsgemäßen Implantat eine um 20% höhere Biegefestigkeit im Punkt des Zementversagens (1. Spannungsabfall) festgestellt.

Der unverstärkte Formkörper derselben Dimensionen aus Magnesium-Calciumphosphat-Zement (Referenzprobe) versagt bei niedriger Verformung vollständig. Das erfindungsgemäße Implantat zeigt dagegen nur einen geringen Kraftabfall und bei weiterer Deformation einen kontinuierlichen Kraftanstieg (Fig. 2).

### Beispiel 8: Mechanische Eigenschaften eines erfindungsgemäßen Implantats mit Magnesium-Calciumphosphat-Zement und einem 2x-Geflecht aus Titandraht mit Stehfäden in Form eines Faserverbundwerkstoffs

Magnesium-Calciumphosphat-Zement wird wie in Beispiel 1 beschrieben bereitgestellt und mit wässriger Ammoniumphosphatlösung angemischt. Die aushärtende Zementpaste wurde in ein 2x-Geflecht aus Reintitanfasern (Faserdurchmesser 0,3 mm) eingebracht. Daraus wurden Formkörper der Abmessung 80x17x4mm³ hergestellt. Der Anteil des Metalls in den Formkörpern betrug 12 Gew.-%). Die Formkörper wurden für 72 h in physiologischer Kochsalzlösung bei 37°C zur Aushärtung gebracht. Die ausgehärteten Formkörper wurden auf einer Universal-Prüfmaschine auf 4-Punkt-Biegefestigkeit untersucht.

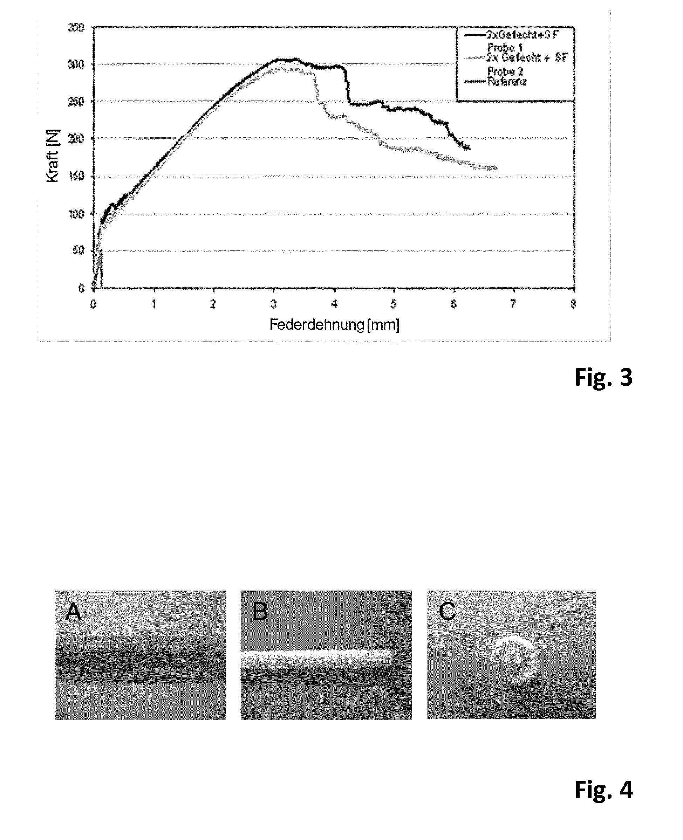

Im Vergleich zu einem unverstärkten Formkörper derselben Dimensionen aus Magnesium-Calciumphosphat-Zement (<20 MPa) wurde bei dem erfindungsgemäßen Implantat mit einem eingebrachten 2x-Geflecht aus Titandraht mit Stehfäden (90 MPa) eine über 4fach höhere Biegefestigkeit ermittelt. Das erfindungsgemäße Implantat zeigt zugleich bei anhaltender Deformation einen kontinuierlichen Kraftanstieg (Fig. 3).

Fig. 3 zeigt den Verlauf der Verformungskurve unter Biegebelastung. Dabei ist eindeutig zu erkennen, dass zur Deformation bzw. zum Versagen der Verbundproben eine deutlich größere Arbeit notwendig ist, als bei dem spröden Bruch der Referenz.

### Beispiel 9: Vergleich der Biegefestigkeit erfindungsgemäßer Implantat in Form von Faserverbundwerkstoffen enthaltend Magnesium-Calciumphosphat-Zement in Verbund mit unterschiedlicher Anzahl an Geflechten aus Titan

Das mechanische Verhalten der Verbundmaterialien wurde in der Hauptbeanspruchungsart, der Biegung ermittelt. Dazu wurde Magnesium-Calciumphosphat-Zement wie in Beispiel 1 beschrieben bereitgestellt und mit wässriger Ammoniumphosphatlösung angemischt. Die aushärtende Zementpaste wurde in ein Gelege aus Reintitanfasern (Faserdurchmesser 0,3 mm) eingebracht, wobei die Gelege unterschiedliche Anzahlen an Geflechten aufweisen. Daraus wurden zylindrische Formkörper der Abmaße l=100mm, d=8mm hergestellt. Der Anteil des Metalls in den Formkörpern betrug je nach Anzahl der Titangeflechte 6 bis 12 Gew.-%). Die Formkörper wurden für 72 h in physiologischer Kochsalzlösung bei 37°C zur Aushärtung gebracht. Die ausgehärteten Formkörper wurden auf einer Universal-Prüfmaschine auf 4-Punkt-Biegefestigkeit untersucht.

In Biegeversuchen an den zylindrischen Verbundproben zeigte sich ein deutlich positiver Einfluss von eingearbeiteten Stehfäden auf die Festigkeit.

Aus den Versuchen (vgl. nachfolgende Tabelle) zeigt sich, dass die Biegefestigkeit von Magnesium-Calciumphosphat-Zement durch das Einbinden von Titangeflechten erhöht werden kann. Die Biegefestigkeit gegenüber einem unverstärkten Magnesium-Calciumphosphat-Zement steigt dabei mit der Anzahl an eingebundenen Titangeflechten von einem 1x-, 2x- zum 3x- und 4x-Geflecht hin an. Der Einsatz eines 4x-Geflechtes im Vergleich zu einem 3x-Geflecht zeigt dabei keine zusätzliche Verstärkungswirkung.

| Probe | Biegefestigkeit [N] |
|---|---|
| Implantat bestehend aus Magnesium-Calciumphosphat-Zement (Referenz) | 55,78 |
| Implantat bestehend aus Magnesium-Calciumphosphat-Zement enthaltend ein 1x-Titangeflecht | 212,00 |
| Implantat bestehend aus Magnesium-Calciumphosphat-Zement enthaltend ein 2x-Titangeflecht | 251,75 |
| Implantat bestehend aus Magnesium-Calciumphosphat-Zement enthaltend ein 3x-Titangeflecht | 375,33 |
| Implantat bestehend aus Magnesium-Calciumphosphat-Zement enthaltend ein 4x-Titangeflecht | 376,75 |

### Beispiel 10: Vergleich der Biegefestigkeit erfindungsgemäßer Implantate mit einem Geflecht aus Titandrähten und/oder Stehfäden im Verbund mit unterschiedlichen Zementzusammensetzungen an Magnesium-Calciumphosphat-Zement in Form eines Faserverbundwerkstoffs

Magnesium-Calciumphosphat-Zement wird wie in Beispiel 1 beschrieben bereitgestellt und mit wässriger Ammoniumphosphatlösung angemischt. Die aushärtende Zementpaste wurde in ein Gelege aus Reintitanfasern (Faserdurchmesser 0,3 mm) eingebracht, wobei die Gelege unterschiedliche Anzahlen an Geflechten und/oder zusätzlich Stehfäden aufweisen. Daraus wurden Formkörper der Abmessung 80x10x6mm³ hergestellt. Der Anteil des Metalls in den Formkörpern betrug je nach Art des eingesetzten Geleges 6-12 Gew.-%). Die Formkörper wurden für 72 h in physiologischer Kochsalzlösung bei 37°C zur Aushärtung gebracht. Die ausgehärteten Formkörper wurden auf einer Universal-Prüfmaschine auf 4-Punkt-Biegefestigkeit untersucht.

Durch den Einsatz von Stehfäden in einem 1x-Geflecht stieg die Biegefestigkeit auf das Vierfache im Vergleich zum Geflechtmaterial ohne Stehfäden an. Auch die Spannungs-Dehnungs-Diagramme der Biegeversuche zeigen deutliche Unterschiede zwischen unverstärkter und verstärkter Probe in der notwendigen Arbeit bis zum Versagen der Materialien.

Weiterhin wurden analog die Biegefestigkeiten an ausgewählten Gelegekonfigurationen, mit denen eine ideale Festigkeitssteigerung bei minimalem Drahtgehalt erreicht werden sollte, gegenüber unverstärkten Implantaten ermittelt. So wurde der Verstärkungseffekt eines 2x-Geflechtes mit Stehfäden mit einem 3x-Geflecht verglichen, um gezielt den Einfluss des Materialgehaltes und zusätzlich eingebrachter Stehfäden auf die mechanischen Eigenschaften des Verbundmaterials zu untersuchen.

| Probe | Biegefestigkeit [N] |
|---|---|
| Implantat bestehend aus Magnesium-Calciumphosphat-Zement (Referenz) | 72,88 |
| Implantat bestehend aus Magnesium-Calciumphosphat-Zement enthaltend ein 2x-Titangeflecht und Stehfäden | 339,25 |
| Implantat bestehend aus Magnesium-Calciumphosphat-Zement enthaltend ein 3x-Titangeflecht | 332,00 |
| Implantat bestehend aus einem polymeren PMMA-Zement | 190,00 |

Erste Bruchereignisse im Zement traten bei etwa einem Drittel der Maximalkraft auf, wobei durch die Drahtverstärkung die weitere Krafteinwirkung aufgenommen wurde, bis die Proben komplett versagten. Mit den beiden Geflechtarten wurden maximale Biegefestigkeiten von ca. 85-95 MPa erreicht, dabei liegen die Mittelwerte für beide Verstärkungsarten auf einem vergleichbaren Niveau.

### Beispiel 11: Fertigung von Formkörpern als Zylinder- und Platten-Bauteile enthaltend Magnesium-Calciumphosphat-Zement

Zur Herstellung der zylindrischen Formkörper wurde eine Metall-Probenform angefertigt. Diese besteht aus zwei Teilen, die halbrund ausgefräst sind und fest zusammengesetzt werden können. An den Enden wird das verwendete Ti-Geflecht fest verankert und kann nach Bedarf gespannt werden. In dieser Probenform wurden zylindrische Formkörper mit den Abmaßen L=200mm, d=10mm hergestellt.

Die Probenpräparation erfolgte mittels einer Spritze und Kanüle auf einem Rüttler in der zylindrischen Form. Es wurden zweilagige-Geflechte mit Stehfäden als Drahtverstärkung verwendet. Der Durchmesser des Titandrahtgeflechtes wurde passend für die Form gewählt (d=10mm), außerdem wurde das Geflecht in der Form so aufgedehnt, dass es seitlich an der Wand anliegt. Die Zementeinfüllung erfolgte von einem offenen Ende her mittels Spritze und einer langen Kanüle unter Rütteln.

Nach Aushärtung über 4 Tage bei 37°C wurden die Enden der Probe begradigt und an beiden Seiten längs verlaufende Grate abgeschliffen. Auf die Gesamtlänge von 200mm verteilte sich die Zementmischung durch Rütteln sehr gut, es waren lediglich sehr kleine Luftblasen an der Probe zu erkennen.

Als weitere Form wurden Platten mit den Abmaßen 2,5x12x80mm³ aus Verbundmaterial hergestellt. Geflechte mit einem Durchmesser von ca. 10mm lassen sich auf eine Größe von ca. 80x12x2,5mm³ (LxBxH) verpressen. Die Verbundproben wurden aus einem zweilagigen Geflecht + Stehfäden hergestellt. Bei der Präparation wurden die Ti-Draht-Geflechte in einem Silikonschlauch unter mechanischer Bewegung auf einem Rüttler mit MgCPC befüllt und anschließend mittels einer hydraulischen Presse zu einer flachen Probe verpresst. Die Probenherstellung in einem durchsichtigen Schlauchmaterial erlaubt die Kontrolle der Zementverteilung während der Befüllung.

Die Proben wurden über 4 Tage bei 37°C ausgehärtet.

Mechanische Kennwerte dieser Osteosynthese-Platten-Bauteile wurden mittels statischer und dynamischer Biegeprüfung ermittelt. Alle Proben, die mit einem 2x-Titangeflecht und Stehfäden verstärkt sind, zeigen einen Zuwachs in der Biegefestigkeit um ca. 400-500% im Vergleich zu den unverstärkten Referenzen (vgl. nachfolgende Tabelle).

| **Probe** | **Biegefestigkeit [MPa]** | |
|---|---|---|
| | unverstärkte Referenz bestehend aus Magnesium-Calciumphosphat-Zement | Implantat bestehend aus Magnesium-Calciumphosphat-Zement enthaltend ein 2x-Titangeflecht und Stehfäden |
| MgCPC-1105231/P 0,6 Zylinder | 48 | 189 |
| MgCPC-120613I/P Zylinder | 23 | 128 |
| MgCPC-120613I/P Platte | 16 | 81 |
| MgCPC-120123C-04A Platte | 15 | 78 |

## Patentansprüche

1. Implantat aus einem Faserverbundwerkstoff, enthaltend:
a) resorbierbaren mineralischen Knochenzement als Matrixmaterial,
b) verstärkende metallische Langfasern und/oder metallische Endlosfasern mit einem Aspektverhältnis von mindestens 100 : 1, in Form mindestens einer gerüstgebenden, die Kontur des Implantates vorformenden Faserstruktur.

2. Implantat gemäß Anspruch 1, **dadurch gekennzeichnet, dass** die gerüstgebenden Faserstrukturen im Implantat in Vorzugsrichtungen ausgerichtet sind und/oder sich im äußeren Bereich des Implantats konzentrieren.

3. Implantat gemäß einem der Ansprüche 1 oder 2, **dadurch gekennzeichnet, dass** die gerüstgebenden Faserstrukturen in Form von Multifilamenten und/oder als Faserhalbzeug aus mindestens einer Lage vorliegen.

4. Implantat gemäß Anspruch 3, **dadurch gekennzeichnet, dass** die Faserstrukturen zwischen den metallischen Fasern Poren einer Größe von 100-2500 *µ*m enthalten.

5. Implantat gemäß einem der Ansprüche 1 bis 4, **dadurch gekennzeichnet, dass** sich mehr als 80 Gew.-% der metallischen Fasern innerhalb eines Bereiches von 0,1 - 5 mm gemessen von der Außenseite des Implantats befinden.

6. Implantat gemäß einem der Ansprüche 1 bis 5, **dadurch gekennzeichnet, dass** die metallischen Langfasern und/oder metallischen Endlosfasern aus einem nichtresorbierbaren Metall bestehen.

7. Implantat gemäß einem der Ansprüche 1 bis 6, **dadurch gekennzeichnet, dass** die metallischen Langfasern und/oder metallischen Endlosfasern aus einem resorbierbaren Metall bestehen.

8. Implantat gemäß einem der Ansprüche 1 bis 7, **dadurch gekennzeichnet, dass** das Implantat eine Druckfestigkeit von > 50 MPa und/oder eine Biegefestigkeit von > 10 MPa aufweist.

9. Implantat gemäß einem der Ansprüche 1 bis 8, **dadurch gekennzeichnet, dass** der mineralische Knochenzement Silikate, Phosphate, Sulfate, Carbonate, Oxide und/oder Hydroxide in Verbindung mit Calciumionen, Magnesiumionen und/oder Strontiumionen enthält.

10. Implantat gemäß einem der Ansprüche 1 bis 9, **dadurch gekennzeichnet, dass** der resorbierbare mineralische Knochenzement in Form einer wasserfreien pastösen Zubereitung vorliegt, wobei in der wasserfreien pastösen Zubereitung mineralisches Knochenzementpulver in einer Trägerflüssigkeit dispergiert ist.

11. Implantat gemäß 10, **dadurch gekennzeichnet, dass** der Gewichtsanteil der Trägerflüssigkeit bezogen auf die Gesamtmasse der wasserfreien pastösen Zubereitung 5 bis 25 Gew.-% beträgt.

12. Implantat gemäß einem der Ansprüche 10 oder 11, **dadurch gekennzeichnet, dass** die wasserfreie pastöse Zubereitung Tenside und/oder einen Abbindebeschleuniger und/oder wasserlösliche Füllstoffe enthält.

13. Implantat gemäß einem der Ansprüche 1 bis 12, **dadurch gekennzeichnet, dass** das Implantat durch Stehfäden verstärkt ist.

14. Verfahren zur Herstellung eines Implantats gemäß einem der Ansprüche 1 bis 13, umfassend die Bereitstellung des Faserverbundwerkstoffs mit den Schritten:
a) Bereitstellung einer pastösen Zubereitung, enthaltend
i. resorbierbares mineralisches Knochenzementpulver,
ii. das in einer Flüssigkeit dispergiert ist,
b) Imprägnierung mehrerer metallischer Langfasern und/oder metallischer Endlosfasern mit einem Aspektverhältnis von mindestens 100 : 1, in Form gerüstgebender Faserstrukturen mit der pastösen Zubereitung.

15. Knochenimplantat bestehend aus mindestens einem Implantat nach Anspruch 1 bis 13 oder enthaltend mindestens ein Implantat nach Anspruch 1 bis 11 in Kombination mit mindestens einem weiteren Formteil, welches fest mit dem Implantat verbunden ist.

16. Set zur Herstellung eines Implantats gemäß einem der Ansprüche 1 bis 11 oder eines Knochenimplantats gemäß Anspruch 15, umfassend die Komponenten:
a) mehrere metallische Langfasern und/oder Endlosfasern mit einem Aspektverhältnis von mindestens 100 : 1, in Form gerüstgebender Faserstrukturen,
b) mindestens eine Knochenzementzubereitung, umfassend resorbierbares mineralisches Knochenzementpulver.

17. Verwendung eines Sets gemäß Anspruch 16 zur Herstellung eines Knochenimplantats.

## Claims

1. Implant made of a fibre composite material, containing:
a) resorbable mineral bone cement as a matrix material,
b) reinforcing, long metallic fibres and/or continuous metallic fibres having an aspect ratio of at least 100:1 in the form of at least one fibre structure which provides a framework and preforms the contour of the implant.

2. Implant according to claim 1, **characterised in that** the framework forming fibre structures are oriented in preferential directions in the implant and/or are concentrated in the outer region of the implant.

3. Implant according to either claim 1 or claim 2, **characterised in that** the framework forming fibre structures are in the form of multifilaments and/or in the form of a semi-finished fibre product made of at least one layer.

4. Implant according to claim 3, **characterised in that** the fibre structures between the metallic fibres contain pores of a size of 100-2500 µm.

5. Implant according to any of claims 1 to 4, **characterised in that** more than 80 wt.% of the metallic fibres are within a range of 0.1-5 mm, measured from the outside of the implant.

6. Implant according to any of claims 1 to 5, **characterised in that** the long metallic fibres and/or continuous metallic fibres consist of a non-resorbable metal.

7. Implant according to any of claims 1 to 6, **characterised in that** the long metallic fibres and/or continuous metallic fibres consist of a resorbable metal.

8. Implant according to any of claims 1 to 7, **characterised in that** the implant has a compressive strength of > 50 MPa and/or a bending strength of > 10 MPa.

9. Implant according to any of claims 1 to 8, **characterised in that** the mineral bone cement contains silicates, phosphates, sulphates, carbonates, oxides and/or hydroxides in combination with calcium ions, magnesium ions and/or strontium ions.

10. Implant according to any of claims 1 to 9, **characterised in that** the resorbable mineral bone cement is in the form of a water-free, pasty preparation, wherein in the water-free, pasty preparation mineral bone cement powder is being dispersed in a carrier fluid.

11. Implant according to claim 10, **characterised in that** the proportion by weight of the carrier fluid is from 5 to 25 wt.% based on the overall mass of the water-free, pasty preparation.

12. Implant according to either claim 10 or claim 11, **characterised in that** the water-free, pasty preparation contains surfactants and/or a setting accelerator and/or water-soluble fillers.

13. Implant according to any of claims 1 to 12, **characterised in that** the implant is reinforced by filler yarn.

14. Method for producing an implant according to any of claims 1 to 13, comprising the provision of the fibre composite material by means of the steps of:
a) providing a pasty preparation containing
i. resorbable mineral bone cement powder,
ii. which is dispersed in a liquid,
b) impregnating a plurality of long metallic fibres and/or continuous metallic fibres, having an aspect ratio of at least 100:1, in the form of framework forming fibre structures, with the pasty preparation,.

15. Bone implant consisting of at least one implant according to claims 1 to 13 or containing at least one implant according to claims 1 to 11 in combination with at least one further moulded part which is rigidly connected to the implant.

16. Set for producing an implant according to any of claims 1 to 11 or a bone implant according to claim 15, comprising the components:
a) a plurality of long metallic fibres and/or continuous fibres having an aspect ratio of at least 100:1 in the form of framework forming fibre structures,
b) at least one bone cement preparation, comprising resorbable mineral bone cement powder.

17. Use of a set according to claim 16 for producing a bone implant.

## Revendications

1. Implant en matériau composite fibreux, contenant :
a) un ciment osseux minéral résorbable en tant que matériau de matrice,
b) des fibres longues métalliques de renforcement et/ou des fibres sans fin métalliques avec un rapport de forme d'au moins 100 : 1, sous la forme d'au moins une structure fibreuse formant l'ossature et préformant le contour de l'implant.

2. Implant selon la revendication 1, **caractérisé en ce que** les structures fibreuses formant l'ossature sont orientées dans l'implant dans des directions préférentielles et/ou se concentrent dans la zone extérieure de l'implant.

3. Implant selon l'une des revendications 1 ou 2, **caractérisé en ce que** les structures fibreuses formant l'ossature se présentent sous la forme de multifilaments et/ou en tant que produit semi-fini fibreux composé d'au moins une couche.

4. Implant selon la revendication 3, **caractérisé en ce que** les structures fibreuses contiennent des pores d'une dimension de 100-2 500 µm entre les fibres métalliques.

5. Implant selon l'une des revendications 1 à 4, **caractérisé en ce que** plus de 80 % en poids des fibres métalliques sont situées à l'intérieur d'une plage de 0,1 - 5 mm, mesurée à partir du côté extérieur de l'implant.

6. Implant selon l'une des revendications 1 à 5, **caractérisé en ce que** les fibres longues métalliques et/ou les fibres sans fin métalliques sont composées d'un métal non résorbable.

7. Implant selon l'une des revendications 1 à 6, **caractérisé en ce que** les fibres longues métalliques et/ou les fibres sans fin métalliques sont composées d'un métal résorbable.

8. Implant selon l'une des revendications 1 à 7, **caractérisé en ce que** l'implant présente une résistance à la pression de > 50 MPa et/ou une résistance à la flexion de > 10 MPa.

9. Implant selon l'une des revendications 1 à 8, **caractérisé en ce que** le ciment osseux minéral contient des silicates, des phosphates, des sulfates, des carbonates, des oxydes et/ou des hydroxydes combinés avec des ions calcium, des ions magnésium et/ou des ions strontium.

10. Implant selon l'une des revendications 1 à 9, **caractérisé en ce que** le ciment osseux minéral résorbable se présente la forme d'une préparation pâteuse exempte d'eau, dans lequel une poudre de ciment osseux minéral est dispersée dans un liquide support dans la préparation pâteuse exempte d'eau.

11. Implant selon la revendication 10, **caractérisé en ce que** la proportion en poids du liquide porteur rapportée à la masse globale de la préparation pâteuse exempte d'eau est comprise entre 5 et 25 % en poids.

12. Implant selon l'une des revendications 10 ou 11, **caractérisé en ce que** la préparation pâteuse exempte d'eau contient des agents tensioactifs et/ou un accélérateur de prise et/ou des charges solubles dans l'eau.

13. Implant selon l'une des revendications 1 à 12, **caractérisé en ce que** l'implant est renforcé par des fils fixes.

14. Procédé de fabrication d'un implant selon l'une des revendications 1 à 13, comprenant la fourniture du matériau composite fibreux, avec les étapes suivantes :
a) fourniture d'une préparation pâteuse, contenant
i. une poudre de ciment osseux minéral résorbable
ii. qui est dispersée dans un liquide,
b) imprégnation de plusieurs fibres longues métalliques et/ou de fibres sans fin métalliques avec un rapport de forme d'au moins 100 : 1, sous la forme de structures fibreuses formant l'ossature avec la préparation pâteuse.

15. Implant osseux, composé d'au moins un implant selon les revendications 1 à 13, ou contenant au moins un implant selon les revendications 1 à 11 en combinaison avec au moins une autre pièce de forme qui est raccordée fixement à l'implant.

16. Kit pour la fabrication d'un implant selon l'une des revendications 1 à 11 ou d'un implant osseux selon la revendication 15,
comprenant les composants suivants :
a) plusieurs fibres longues et/ou fibres sans fin métalliques avec un rapport de forme d'au moins 100 : 1, sous la forme de structures fibreuses formant l'ossature,
b) au moins une préparation de ciment osseux, comprenant une poudre de ciment osseux minéral résorbable.

17. Utilisation d'un kit selon la revendication 16 pour la fabrication d'un implant osseux.
